# FASCICULE DE BREVET EUROPEEN

(11) **EP 1 762 203 B1**
(45) Date de publication et mention de la délivrance du brevet: **21.10.2015**
(21) Numéro de dépôt: 06012485.6
(22) Date de dépôt: 17.06.2006
(51) Int. Cl.: A61F 2/46, G06F 19/00

(54) **Procédé assisté par ordinateur pour sélectionner une stratégie optimale de remplacement d'une prothèse fémorale**
Rechnergestütztes Verfahren zum Auswählen einer optimalen Femurprothese-Ersatz-Strategie
Computer-aided process for selecting an optimal femoral prosthesis replacement strategy

(30) Priorité: 29.06.2005 FR 0506756
(43) Date de publication de la demande: 14.03.2007
(73) Titulaire: Zimmer France SAS, 25461 Etupes Cedex (FR); Le Beguec, Pierre, 35000 Rennes (FR)
(72) Inventeur: Le Beguec, Pierre, 25000 Besancon (FR)
(74) Mandataire: Mays, Julie

(56) Documents cités:
- WO-A-03/039377
- US-A- 5 408 409
- US-A- 5 824 085
- US-A1- 2005 038 338
- US-B1- 6 205 411

## Description

L'invention concerne, de façon générale, les techniques d'optimisation de processus concrets à caractère complexe.

Plus précisément, l'invention concerne un procédé tel que défini dans la revendication 1 pour sélectionner, parmi un ensemble de stratégies utilisables connues, une stratégie de remplacement d'une prothèse fémorale.

On connaît déjà, par la demande de brevet US 2005/0038338, un procédé assisté par ordinateur pour sélectionner, parmi plusieurs prothèses fémorales possibles, la prothèse qui, par ses dimensions ou sa conformation, est la plus adaptée à un patient donné.

En dehors des aspects chirurgicaux, qui sont totalement étrangers à la présente invention, le remplacement de prothèses fémorales fait intervenir des problèmes de mécanique et de résistance des matériaux dont la résolution est complexe.

On connaît en effet aujourd'hui différents types de prothèses fémorales et un certain nombre de stratégies de remplacement de ces prothèses, plus ou moins adaptées au type de prothèses utilisées.

Avant tout acte chirurgical, et après le diagnostic, lui aussi totalement étranger à la présente invention, que chaque praticien doit faire sur chaque nouveau cas qui lui est soumis, et au terme duquel il prend la décision de remplacer, ou non, la prothèse défaillante en fonction notamment de l'état de santé du patient concerné, se pose donc la question, en cas de décision de remplacement de la prothèse, du choix de la stratégie à mettre en oeuvre pour ce remplacement, cette stratégie dépendant de considérations purement techniques, en particulier de mécanique et de résistance des matériaux.

On connaît, par le brevet US 5 824 085, un procédé assisté par ordinateur et conforme au préambule de la revendication 1 pour sélectionner, parmi un ensemble de stratégies possibles, une stratégie de remplacement optimale d'une prothèse fémorale, ce procédé reposant sur des considérations géométriques et l'utilisation de références théoriques en résistance des matéiraux.

Néanmoins, le choix optimal d'une telle prothèse est aujourd'hui le plus souvent réalisé par le praticien lui-même, éventuellement sur les conseils d'un prothésiste, de façon tout à fait empirique et donc sans autre garantie d'optimisation que celle que peut laisser espérer l'expérience du praticien ou celle du prothésiste.

Dans ce contexte, l'invention a pour but de proposer un procédé permettant, par une rationalisation de ce choix et le recours à l'ordinateur, d'identifier la stratégie optimale de remplacement d'une prothèse fémorale.

L'un des critères de classification est de préférence constitué par la forme du fémur, dont les différentes valeurs ou instances particulières sont constituées par différents types de forme.

Un deuxième critère de classification est de préférence constitué par les altérations osseuses du fémur, dont les différentes valeurs ou instances particulières sont constituées par différents stades d'altérations.

Un troisième critère de classification est de préférence constitué par la densité osseuse du fémur, dont les différentes valeurs ou instances particulières sont constituées par différents stades d'ostéoporose.

Un quatrième critère de classification est de préférence constitué par le mode de fixation de la prothèse existante, ce critère prenant au moins l'une ou l'autre de deux valeurs selon que cette prothèse est, ou non, fixée au moyen d'un ciment.

Il est par ailleurs décrit un programme d'ordinateur permettant, lorsqu'il est utilisé sur un ordinateur, de sélectionner, parmi un ensemble de stratégies utilisables connues, une stratégie de remplacement optimale d'une prothèse fémorale pour un nouveau cas de remplacement, l'ordinateur comportant une mémoire dans laquelle sont classées plusieurs configurations connues dont chacune est définie par des valeurs ou instances particulières de différents critères pré-établis, et à chacune desquelles est associée une stratégie optimale de remplacement, ce programme comprenant un module de saisie des différentes valeurs ou instances particulières que prennent lesdits critères dans la configuration que constitue le nouveau cas, un module de recherche pour identifier la stratégie optimale associée à la configuration que constitue ce nouveau cas, et un module d'affichage pour afficher un descriptif de ladite stratégie optimale.

D'autres caractéristiques et avantages de l'invention ressortiront clairement de la description qui en est faite ci-après, à titre indicatif et nullement limitatif, en référence aux dessins annexés, dans lesquels :
- la figure 1 est une représentation schématique d'une liste de critères de classification;
- la figure 2 est une représentation schématique d'un catalogue de configurations;
- la figure 3 est une représentation schématique d'une table de correspondance associant des configurations à des stratégies optimales; et
- la figure 4 est une représentation schématique des modules d'un programme d'ordinateur conforme à l'invention et de leur fonction.

Par convention d'écriture, les lettres n, x, y, z, i, j, k, q et p représentent des nombres entiers dont la valeur n'est pas spécifiée a priori.

Comme annoncé précédemment, l'invention concerne un procédé assisté par ordinateur permettant de sélectionner, de façon automatique, une stratégie de remplacement optimale SRT OP d'une prothèse fémorale parmi un ensemble de stratégies utilisables connues STR1 à STRp.

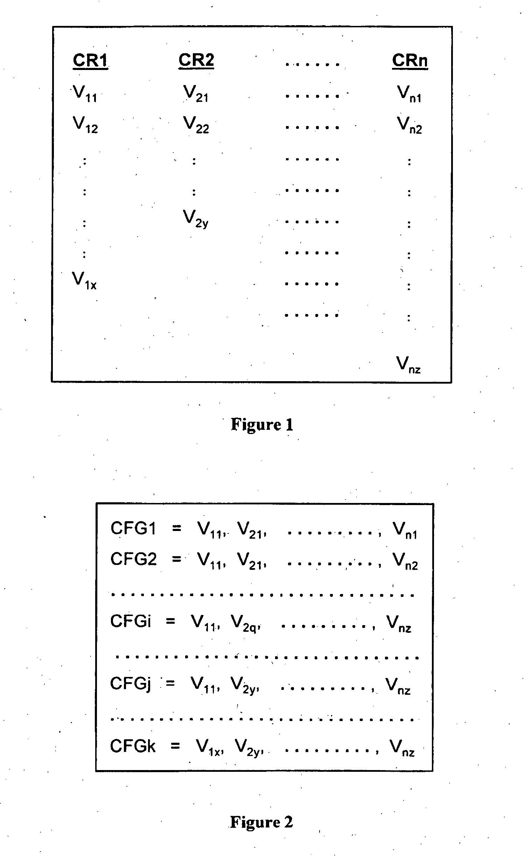

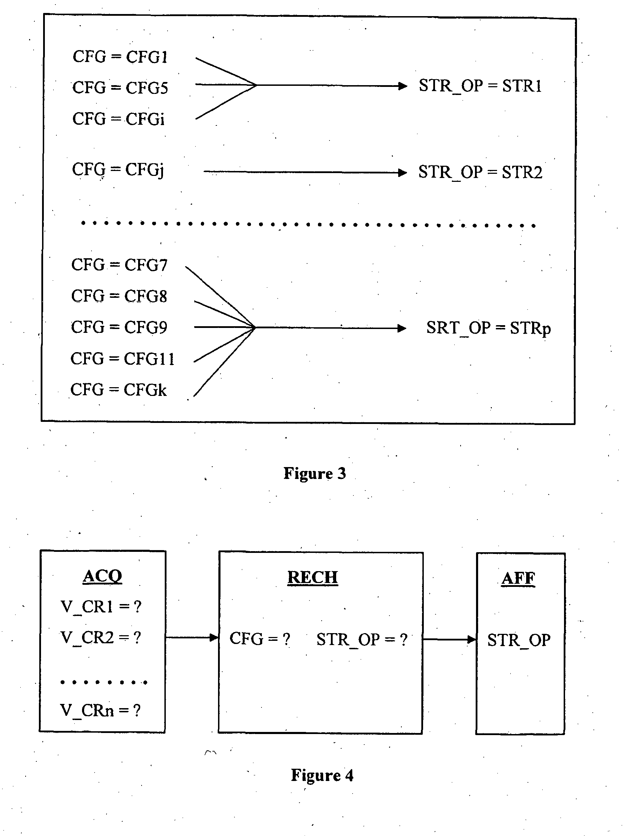

Ce procédé comprend essentiellement deux phases, à savoir une phase préliminaire de collecte de données schématisée sur les figures 1 à 3, et une phase d'exploitation appliquée à chaque nouveau cas de remplacement de prothèse fémorale et schématisée sur la figure 4.

La phase préliminaire de collecte comprend tout d'abord une opération consistant à recenser, sur des cas réels de remplacement de prothèses fémorales, divers critères CR1 à CRn de classification de ces cas, ces critères étant établis par observation de ces cas préalablement au remplacement de prothèse auquel ils ont donné lieu.

Chaque critère est choisi de manière à pouvoir faire l'objet d'une évaluation objective, par exemple d'une quantification, et correspond à un paramètre physique prenant, sur les différents cas observés, des valeurs ou instances particulières différentes, telles que V11 à V1x pour le critère CR1, V21 à V2y pour le critère CR2, et Vn1 à Vnz pour le critère CRn (figure 1).

L'un des critères de classification, par exemple CR1, peut avantageusement être constitué par la forme du fémur.

Le fémur peut présenter différents types de formes et notamment être droit ou courbe de face, et droit ou courbe de profil.

La courbure peut être évaluée de façon objective par comparaison avec un rayon de courbure limite prédéterminé, le fémur étant considéré comme droit ou courbe selon que son rayon de courbure est supérieur ou au plus égal à ce rayon de courbure limite.

On peut donc choisir de donner au critère CR1 de forme du fémur par exemple soit deux valeurs ou instances particulières, à savoir droit ou courbe de face, ou droit ou courbe de profil, soit quatre valeurs ou instances particulières, à savoir droit de face et de profil, droit de face et courbe de profil, courbe de face et droit de profil et courbe de face et de profil.

En se référant à la notation dans laquelle le critère CR1 prend les valeurs V11 à V1x, le nombre "x" est donc égal à 2 ou 4 dans l'exemple ci-dessus.

Un autre critère de classification, par exemple CR2, peut avantageusement être constitué par la densité osseuse du fémur, éventuellement mesurée par ostéodensitométrie.

On peut alors choisir de découper en trois plages les différentes valeurs possibles de densités osseuses susceptibles d'être rencontrées, ces plages correspondant à différents stades d'ostéoporose.

En se référant à la notation dans laquelle le critère CR2 prend les valeurs V21 à V2y, le nombre "y" est donc égal à 3 dans cet exemple.

Un autre critère de classification, par exemple CR3, peut avantageusement être constitué par les altérations osseuses affectant le fémur.

Il est en effet possible de distinguer, dans l'apparition d'altérations osseuses sur le fémur, quatre stades différents, de sorte que le critère CR3 pourra prendre les valeurs V31 à V34.

Un autre critère de classification, par exemple CRn, peut être constitué par le mode de fixation de la prothèse existante, ce critère prenant l'une ou l'autre de deux valeurs selon que cette prothèse est ou non fixée au moyen d'un ciment.

En se référant à la notation dans laquelle le critère CRn prend les valeurs Vn1 à Vnz, le nombre "z" est donc égal à 2 dans cet exemple.

Les critères de classification CR1 à CRn ayant été recensés, la phase préliminaire de collecte se poursuit par une opération consistant à établir un catalogue des différentes configurations rencontrées sur les cas observés et notées CFG1, CFG2, ..., CFGi, ..., CFGj, ..., CFGk (figure 2).

Par "configuration" on entend ici une collection particulière de valeurs ou instances particulières respectives des différents critères CR1 à CRn, deux configurations différentes quelconques se distinguant donc l'une de l'autre par au moins une valeur ou instance particulière de l'un au moins des critères CR1 à CRn.

Les cas réels observés ayant ainsi été regroupés en configurations, et chaque cas réel ayant été traité par l'une des stratégies de remplacement connues telles que STR1, STR2, ou STRp, la phase préliminaire de collecte consiste alors à sélectionner, pour chaque configuration, celle des stratégies utilisées pour les cas réels appartenant à cette configuration qui a statistiquement donné les meilleurs résultats, essentiellement en termes de tenue mécanique et de durabilité, cette stratégie étant donc considérée comme la stratégie optimale STR_OP pour cette configuration (figure 3).

La dernière opération de la phase préliminaire de collecte consiste à enregistrer dans la mémoire de l'ordinateur un lien entre chaque configuration rencontrée et la stratégie qui s'est avérée, sur la base des cas réels observés, être la stratégie optimale pour cette configuration.

Par exemple (figure 3), dans les cas où la configuration rencontrée CFG était constituée par la configuration CFG1, ou par la configuration CFG5, ou encore par la configuration CFGi, alors la stratégie optimale STR OP s'est avérée être constituée par la stratégie STR1.

Une fois ces données enregistrées dans la mémoire de l'ordinateur, la phase d'exploitation du procédé peut être mise en oeuvre pour chaque nouveau cas rencontré.

Pour ce faire, l'ordinateur est doté d'un programme comprenant au moins un module de saisie ACQ, un module de recherche RECH et un module d'affichage AFF.

Le module de saisie permet à l'utilisateur d'indiquer à l'ordinateur les valeurs ou instances particulières, telles que V_CR1, V_CR2, ... , V_CRn que prennent les différents critères CR1, CR2, ... , CRn pour le nouveau cas rencontré.

Le module de recherche RECH a d'abord pour fonction de retrouver, dans le catalogue de configurations CFG1 à CFGk (figure 2), la configuration CFG qui est définie par ces valeurs ou instances particulières V_CR1 à V_CRn.

Le module de recherche RECH a par ailleurs pour fonction d'extraire de la mémoire de l'ordinateur, en utilisant les liens qui y ont été enregistrés et qu'illustre la figure 3, la stratégie de remplacement optimale STR_OP qui a été associée à cette configuration CFG.

Enfin, le module d'affichage AFF affiche un descriptif identifiant la stratégie optimale STR_OP recommandée pour ce nouveau cas.

## Revendications

1. Procédé assisté par ordinateur pour sélectionner, parmi un ensemble de stratégies utilisables connues (STR1 à STRp), une stratégie de remplacement-optimale (SRT_OP) d'une prothèse fémorale, ce procédé comprenant une phase préliminaire de collecte de données et une phase d'exploitation appliquée à chaque nouveau cas de remplacement de prothèse fémorale, la phase préliminaire comprenant au moins les opérations consistant à : recenser, sur des cas réels de remplacement de prothèses fémorales observés préalablement à ce remplacement, divers critères (CR1 à CRn) de classification de ces cas, chacun de ces critères étant susceptible de faire l'objet d'une évaluation objective, telle qu'une quantification, et d'adopter, d'un cas à l'autre, des valeurs ou instances particulières différentes (V11-V1x; V21-V2y; Vn1-Vnz); établir un catalogue des différentes configurations (CFG1-CFGk) rencontrées sur les cas observés, chaque configuration (CFG1-CFGk) étant définie par une collection particulière de valeurs ou instances particulières respectives des différents critères (CR1 à CRn); identifier, pour chaque configuration rencontrée, une stratégie de remplacement optimale (SRT_OP) définie comme celle des stratégies qui, parmi une pluralité de stratégies de remplacement (STR1-STRp) effectivement utilisées dans les cas réels recensés et répondant à cette configuration, a conduit au moins statistiquement aux résultats les plus satisfaisants; et enregistrer dans la mémoire de l'ordinateur la stratégie de remplacement optimale identifiée pour chaque configuration rencontrée, la phase d'exploitation du procédé étant mise en oeuvre, pour chaque nouveau cas, postérieurement au diagnostic de ce nouveau cas et antérieurement à toute intervention chirurgicale pour ce nouveau cas, et comprenant au moins les opérations consistant à : indiquer à l'ordinateur les valeurs ou instances particulières (V_CR1-V_CRn) que prennent les différents critères pour ce nouveau cas; retrouver, dans le catalogue de configurations (CFG1-CFGk), la configuration définie par ces valeurs ou instances particulières (V_CR1-V_CRn); et extraire de la mémoire de l'ordinateur la stratégie de remplacement optimale (STR_OP) qui a été associée à cette configuration.

2. Procédé suivant la revendication 1, **caractérisé en ce qu'**un des critères de classification est constitué par la forme du fémur, dont les différentes valeurs ou instances particulières sont constituées par différents types de forme.

3. Procédé suivant la revendication 1 ou 2, **caractérisé en ce qu'**un des critères de classification est constitué par les altérations osseuses du fémur, dont les différentes valeurs ou instances particulières sont constituées par différents stades d'altérations.

4. Procédé suivant l'une quelconque des revendications précédentes, **caractérisé en ce qu'**un des critères de classification est constitué par la densité osseuse du fémur, dont les différentes valeurs ou instances particulières sont constituées par différents stades d'ostéoporose.

5. Procédé suivant l'une quelconque des revendications précédentes, **caractérisé en ce qu'**un des critères de classification est constitué par le mode de fixation de la prothèse existante, ce critère prenant au moins l'une ou l'autre de deux valeurs selon que cette prothèse est, ou non, fixée au moyen d'un ciment.

## Patentansprüche

1. Computergestütztes Verfahren zum Auswählen einer optimalen Strategie (SRT_OP) zum Ersetzen einer Oberschenkelknochen-Prothese aus einer Einheit von bekannten verwendbaren Strategien (STR1 bis STRp), wobei dieses Verfahren eine Vorphase der Sammlung von Daten und eine Phase der Auswertung umfasst, die auf jeden neuen Fall des Ersatzes einer Oberschenkelknochen-Prothese angewendet wird, wobei die Vorphase mindestens die folgenden Schritte umfasst: Erheben verschiedener Kriterien (CR1 bis CRn) anhand realer Fälle des Ersatzes von Oberschenkelknochen-Prothesen, die vor diesem Ersatz beobachtet werden, zur Klassifizierung dieser Fälle, wobei jedes dieser Kriterien Gegenstand einer objektiven Auswertung, wie einer Quantifizierung, sein kann und von einem Fall zum anderen verschiedene spezielle Werte oder Instanzen (V11-V1x; V21-V2y; Vn1-Vnz) annehmen kann; Erstellen eines Katalogs der verschiedenen Konfigurationen (CFG1-CFGk), die an den beobachteten Fällen vorgefunden werden, wobei jede Konfiguration (CFG1-CFGk) durch eine spezielle Sammlung von jeweiligen speziellen Werten oder Instanzen der verschiedenen Kriterien (CR1 bis CRn) definiert ist; Identifizieren einer optimalen Ersatzstrategie (SRT_OP) für jede vorgefundene Konfiguration, die als diejenige der Strategien definiert ist, die aus einer Vielzahl von Ersatzstrategien (STR1-STRp), die in den erhobenen realen Fällen effektiv verwendet wurden und diese Konfiguration erfüllen, wenigstens statistisch zu den am meisten zufriedenstellenden Ergebnissen geführt hat; und Aufzeichnen der für jede vorgefundene Konfiguration identifizierten optimalen Ersatzstrategie im Speicher des Rechners, wobei die Auswertungsphase für jeden neuen Fall nach der Diagnose dieses neuen Falles und vor einem beliebigen chirurgischen Eingriff für diesen neuen Fall durchgeführt wird und mindestens folgende Schritte umfasst: Anzeigen am Rechner der speziellen Werte oder Instanzen (V_CR1_V_CRn), welche die verschiedenen Kriterien für diesen neuen Fall nehmen; Auffinden in dem Konfigurationskatalog (CFG1-CFGk) der durch diese speziellen Werte oder Instanzen (V_CR1_V_CRn) definierten Konfiguration; und Extrahieren der optimalen Ersatzstrategie (STR_OP), die dieser Konfiguration zugeordnet ist, aus dem Speicher des Rechners.

2. Verfahren nach Anspruch 1, **dadurch gekennzeichnet, dass** eines der Klassifizierungskriterien von der Form des Oberschenkelknochens gebildet ist, deren verschiedene spezielle Werte oder Instanzen von verschiedenen Formtypen gebildet sind.

3. Verfahren nach Anspruch 1 oder 2, **dadurch gekennzeichnet, dass** eines der Klassifizierungskriterien von den Knochenveränderungen des Oberschenkelknochens gebildet ist, deren verschiedene spezielle Werte oder Instanzen von verschiedenen Veränderungsstadien gebildet sind.

4. Verfahren nach einem der vorhergehenden Ansprüche, **dadurch gekennzeichnet, dass** eines der Klassifizierungskriterien von der Knochendichte des Oberschenkelknochens gebildet ist, deren verschiedene spezielle Werte oder Instanzen von verschiedenen Osteoporosestadien gebildet sind.

5. Verfahren nach einem der vorhergehenden Ansprüche, **dadurch gekennzeichnet, dass** eines der Klassifizierungskriterien von der Art der Befestigung der vorhandenen Prothese gebildet ist, wobei dieses Kriterium wenigstens den einen oder den anderen der zwei Werte nimmt, je nachdem, ob diese Prothese mit Hilfe von Zement oder nicht mit Hilfe von Zement befestigt ist.

## Claims

1. Computer-aided method for selecting, from a set of known usable strategies (STR1 to STRp), an optimum strategy (STR_OP) for replacing a femoral prosthesis, said method comprising a preliminary phase of collecting data and a processing phase which is applied to each new femoral prosthesis replacement case, the preliminary phase comprising at least the operations consisting in identifying, in real femoral prosthesis replacement cases observed prior to this replacement, various criteria (CR1 to CRn) for classifying said cases, each of these criteria being capable of being subject to an objective evaluation, such as a quantification, and of assuming, from one case to another, different particular values or instances (V11-V1x; V21-V2y; Vn1-Vnz); creating a catalogue of the different configurations (CFG1-CFGk) found in the observed cases, each configuration (CFG1-CFGk) being defined by a particular collection of respective particular values or instances of the different criteria (CR1 to CRn); identifying, for each configuration encountered, an optimum replacement strategy (SRT_OP) defined as being that strategy which has led to the most satisfying results, at least statistically, among a plurality of replacement strategies (STR1-STRp) which were used effectively in the real cases surveyed and fit said configuration; and recording, in the computer memory, the optimum replacement strategy identified for each configuration encountered, the processing phase of the method being carried out, for each new case, following the diagnosis of this new case and prior to any surgical intervention for this new case, and comprising at least the operations consisting in indicating to the computer the particular values or instances (V_CR1 -V_CRn) assumed by the different criteria for this new case; finding, in the catalogue of configurations (CFG1-CFGk), the configuration defined by these particular values or instances (V_CR1-V_CRn); and extracting from the computer memory the optimum replacement strategy (STR_OP) which has been associated with this configuration.

2. Method according to claim 1, **characterised in that** one of the classification criteria is represented by the shape of the femur, the different particular values or instances of which are represented by different shapes.

3. Method according to either claim 1 or claim 2, **characterised in that** one of the classification criteria is represented by the bone changes to the femur, the different particular values or instances of which are represented by different stages of change.

4. Method according to any of the preceding claims, **characterised in that** one of the classification criteria is represented by the bone density of the femur, the different particular values or instances of which are represented by different stages of osteoporosis.

5. Method according to any of the preceding claims, **characterised in that** one of the classification criteria is represented by the manner in which the existing prosthesis is attached, this criteria assuming at least one or the other of two values, according to which this prosthesis is attached or not attached by means of a cement.
